(19) Europäisches Patentamt
European Patent Office
Office européen des brevets

(11) Numéro de publication : **0 429 360 B1**

# (12) FASCICULE DE BREVET EUROPEEN

(45) Date de publication du fascicule du brevet : **13.10.93 Bulletin 93/41**

(51) Int. Cl.$^5$ : **A61K 31/505**

(21) Numéro de dépôt : **90403284.4**

(22) Date de dépôt : **21.11.90**

## (54) Inhibition du syndrome d'abstinence.

(30) Priorité : **22.11.89 FR 8915316**

(43) Date de publication de la demande : **29.05.91 Bulletin 91/22**

(45) Mention de la délivrance du brevet : **13.10.93 Bulletin 93/41**

(84) Etats contractants désignés : **AT BE CH DE DK FR GB GR IT LI LU NL SE**

(56) Documents cités :
**EP-A- 0 285 008**
**EP-A- 0 382 637**

(73) Titulaire : **LABORATORIOS DEL DR. ESTEVE, S.A.**
**Av. Mare de Deu de Montserrat, 221**
**E-08026 Barcelona (ES)**

(72) Inventeur : **Frigola-Constansa, Jordi**
**Av. Diagonal, 299 at.1a**
**E-08013 Barcelone (ES)**
Inventeur : **Pares-Corominas, Juan**
**Padilla, 349, 3o 3a**
**E-08025 Barcelone (ES)**

(74) Mandataire : **Ahner, Francis et al**
**CABINET REGIMBEAU, 26, avenue Kléber**
**F-75116 Paris (FR)**

**EP 0 429 360 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation des dérivés de 1H-azole-(ω-(4-(2-pyrimidinyl)-1-pipérazinyl)-alkyl) ainsi que de leurs sels physiologiquement acceptables, pour la fabrication de médicaments destinés au traitement des troubles associés au syndrome d'abstinence induit par la suppression de benzodiazépines en particulier de Diazépan, de la cocaïne, de l'alcool et/ou de la nicotine.

Alors que l'ensemble des anxiolytiques connus se sont toujours révélés incapables d'inhiber le syndrome d'abstinence, il a été constaté de façon totalement surprenante que certains dérivés de 1H-azole-(ω-(4-(2-pyrimidinyl)-1-pipérazinyl)-alkyl) étaient très actifs pour traiter les troubles associés à un tel syndrome.

L'état de la technique antérieure peut en outre être illustrée par le brevet EP-A-0 382 637, qui décrit des dérivés de pyrimidinyl-piperazinyl-alkyl-azole présentés comme dotés d'une activité anxiolytique et/ou tranquillisante.

Le brevet EP-A-0 285 008 enseigne l'utilisation de la buspirone pour le traitement des troubles associés au syndrome d'abstinence induit par la suppression de l'alcool. Il s'agit là de composés qui, par rapport à ceux de la présente invention, d'une part présentent une structure différente et d'autre part une réponse pharmacologique très inférieure.

Plus précisément, l'invention concerne l'utilisation des dérivés de formule générale I

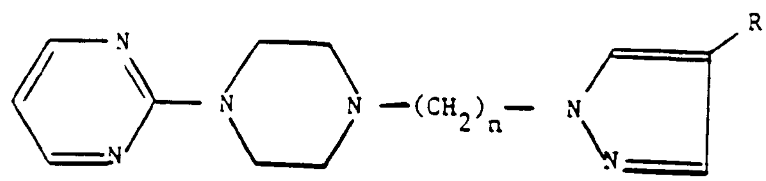

(I)

dans laquelle:
n peut avoir les valeurs 1 à 6, et
R représente un atome d'hydrogène, un halogène, un radical alkyle inférieur en $C_1$ à $C_4$, un radical pyrolyle, un radical sulfonique, un radical N-diméthyl-sulfamoyle, un radical nitro, un radical alcoxy inférieur en $C_1$ à $C_4$, un radical cyano, un radical carboxylate d'alkyle inférieur en $C_1$ à $C_4$, un radical phényle ou phényle substitué par un atome de chlore ou par un groupe méthoxy, un radical amino ou amino substitué, de formule

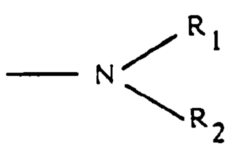

dans laquelle
$R_1$ et $R_2$, identiques ou différents, représentent un atome d'hydrogène, un radical alkyle, un radical alkylcarboxy, un radical phénylcarboxy, un radical alkylsulfonyle, un radical phénylsulfonyle ou un radical tolylsulfonyle, les fragments alkyle de ces radicaux contenant de 1 à 4 atomes de carbone,
et leurs sels thérapeutiquement acceptables,
pour la fabrication de médicaments destinés au traitement des troubles associés au syndrome d'abstinence induit par la suppression de benzodiazépines telles que le Diazépam, de la cocaïne, de l'alcool et/ou de la nicotine.

Plus particulièrement, les dérivés de formule générale I seront choisis parmi:
1H-pyrazole-1-(4-(4-(2-pyrimidinyl)-1pipérazinyl)-butyl), (compsé 1)
1H-pyrazole-4-méthyl-1-(4-(4-(2-pyrimidinyl)-1-pipérazinyl)-butyl),
1H-pyrazole-4-nitro-1-(4-(4-(2-pyrimidynil)-1-pipérazinyl)-butyl),
1H-pyrazole-4-chloro-1-(4-(4-(2-pyrimidynil)-1-pipérazinyl)-butyl), (composé 4)
1H-pyrazole-4-carboxylate d'éthyle-1-(4-(4-(2-pyrimidinyl)-1-pipérazinyl)-butyl),
1H-pyrazole-4-bromo-1-(4-(4-(2-pyrimidinyl)-1-pipérazinyl)-butyl),
1H-pyrazole-4-cyano-1-(4-(4-(2-pyrimidinyl)-1-pipérazinyl)-butyl),
1H-pyrazole-4-fluoro-1-(4-(4-(2-pyrimidinyl)-1-pipérazinyl)-butyl),

1H-pyrazole-4-méthoxy-1-(4-(4-(2-pyrimidinyl)-1-pipérazinyl)-butyl),
1H-pyrazole-4-amino-1-(4-(4-(2-pyrimidinyl)-1-pipérazinyl)-butyl),
1H-pyrazole-4-méthylsulfonamido-1-(4-(4-(2-pyrimidinyl)-1-pipérazinyl)-butyl), (composé (11)
1H-pyrazole-4-benzamido-1-(4-(4-(2-pyrimidinyl)-1-pipérazinyl)-butyl),
1H-pyrazole-4-acétamido-1-(4-(4-(2-pyrimidinyl)-1-pipérazinyl)-butyl),
1H-pyrazole-4-(2-butyl)amino-1-(4-(4-(2-pyrimidinyl)-1-pipérazinyl)- butyl).
1H-pyrazole-4-(4-méthoxyphényl)-1-(4-(4-(2-pyrimidinyl)-1-pipirazinyl)-butyl),
1H-pyrazole-4-(4-chlorophényl)-1-(4-(4-(2-pyrimidinyl)-1-pipérazinyl)-butyl),
1H-pyrazole-4-(1-pyrrolyl)-1-(4-(4-(2-pyrimidinyl)-1-pipérazinyl)-butyl),
1H-pyrazole-4-phényl-1-(4-(4-(2-pyrimidinyl)-1-pipérazinyl)-butyl),
1H-pyrazole-4-phénylsulfonamido-1-(4-(4-(2-pyrimidinyl)-1-pipérazinyl)-butyl),
1H-pyrazole-4-(4-méthylbenzene)sulfonamido-1-(4-(4-(2-pyrimidinyl)-1-pipérazinyl)-butyl),
1H-pyrazole-4-butylsulfonamido-1-(4-(4-(2-pyrimidinyl)-1-pipérazinyl)-butyl),
1H-pyrazole-4-propylsulfonamido-1-(4-(4-(2-pyrimidinyl)-1-pipérazinyl)-butyl),
1H-pyrazole-4-éthylsulfonamido-1-(4-(4-(2-pyrimidinyl)-1-pipérazinyl)-butyl),
1H-pyrazole-4-N-diméthyl-sulfamoyle-1-(4-(4-(2-pyrimidinyl)-1-pipérazinyl)- butyl),
1H-pyrazole-4-sulfonique-1-(4-(4-(2-pyrimidinyl)-1-pipérazinyl)-butyl),
1H-pyrazole-4-chloro-1-(4-(4-(2-pyrimidinyl)-1-pipérazinyl)-butyl)hydrochlorure,
1H-pyrazole-4-chloro-1-(4-(4-(2-pyrimidinyl)-1-pipérazinyl)-butyl)dihidrochlorure,

Des précisions physico-chimiques relatives à ces dérivés ainsi que leur procédé de fabrication se trouvent décrits dans EP-A-0 382 637 qui, à ce titre, est incorporé à la présente description par référence.

Etude de l'activité anxiolitique ou anxiogénique chez la souris

On utilise le test de la boîte claire / boîte obscure, décrit par B. Costall et al. (J. Phar. Pharmacol., 1988, 40 : 494-500). On place la souris dans la zone claire d'une boîte divisée en deux compartiments, un très illuminé, boîte claire, et l'autre peu illuminé, boîte obscure.

1) On compte le nombre de fois que la souris se dresse sur les pattes arrières dans chaque compartiment pendant 5 minutes. (voir colonne 1 du tableau ci-après)

2) L'activité dans chaque compartiment est donnée par le comptage du nombre de croisements par les carreaux qui constituent les divisions de chaque compartiment. (voir colonne 2 du tableau ci-après)

3) On mesure le temps passé dans la boîte obscure pendant les 5 minutes du comptage. (voir colonne 3 du tableau ci-après)

4) On détermine la latence initiale, c'est-à-dire le temps passé depuis que l'on place l'animal dans la boîte claire, au début de l'essai, jusqu'à son entrée dans la boîte obscure. (voir colonne 4 du tableau ci-après)

On détermine le comportement anxiolitique ou anxiogénique des souris au cours des différentes périodes du traitement, en le comparant toujours avec celui d'un groupe d'animaux de contrôle n'ayant subi aucun traitement.

Traitements et schéma expérimental

1) Dépendance d'un traitement déterminé (Diazépam, cocaine. alcool ou nicotine) moyennant l'administration quotidienne pendant une période de 7 ou de 14 jours. Ce traitement provoque une réponse anxiolitique (l'activité et le temps de présence dans la boîte claire augmentent).

Dosages :

| | |
|---|---|
| Diazépam : | 10 mg/kg i.p., 2 fois par jour pendant 7 jours. |
| Cocaïne : | 1 mg/kg i.p., pendant 14 jours. |
| Alcool : | administré au 8% poids/volume dans l'eau de boisson pendant 14 jours. |
| Nicotine : | 0.1 mg/kg i.p., 2 fois par jour pendant 14 jours. |

2) Arrêt du traitement, ce qui en 24 heures provoque un syndrome d'abstinence, qui se manifeste comme une réponse anxiogénique (augmente l'activité et la présence dans la boîte obscure).

3) D'autres groupes différents reçoivent en plus du Diazépam, cocaine, alcool ou nicotine, un traitement concomitant avec des produits objets de la présente invention, et à titre de comparaison avec la Buspirone ou l'Ipsapirone. On retire également à ces groupes le traitement au Diazépam, cocaïne, alcool ou nicotine et on observe aussi la réponse au bout de 24 heures.

Les produits décrits et les doses testées ont été :

1H-pyrazole-4-chloro-1-(4-(4-(2-pyrimidinyl)-1-pipérazinyl)-butyl)
composé 4 : 0.5 mg/kg i.p., 2 fois par jour.

1H-pyrazole-1-(4-(4-(2-pyrimidinyl)-1-pipérazinyl)-butyl)
composé 1 : 0.5 mg/kg i.p., 2 fois par jour.
1H-pyrazole-4-méthylsulfonamido-1-(4-(4-(2-pyrimidinyl)-1-pipérazinyl)-butyl)
composé 11 : 1 mg/kg i.p., 2 fois par jour (face à Diazépam, alcool et nicotine) et 0.5 mg/kg i.p., 2 fois par jour (face à cocaïne).

Les résultats observés ont été consignés dans les tableaux ci-après.

Les réponses obtenues avec les produits objets de la présente invention ont été les suivantes :

Les composés 1 et 4 inhibent le syndrome d'abstinence, qui se manifeste comme une réponse anxiogénique, induit par le Diazépam, la cocaïne, l'alcool et la nicotine, et de plus ils maintiennent une réponse anxiolitique significative lorsqu'on arrête l'administration du Diazépam, de la cocaïne, de l'alcool ou de la nicotine.

Le composé 11 inhibent le syndrome d'abstinence, qui se manifeste comme une réponse anxiogénique, induit par le Diazépam, l'alcool et la nicotine, et de plus il maintient une réponse anxiolitique significative lorsqu'on arrête l'administration du Diazépam et de l'alcool.

La Buspirone maintient le syndrome d'abstinence, qui se manifeste par une réponse anxiogénique, induit par le Diazépam et la cocaïne. Cependant, lorsqu'on arrête le traitement avec l'alcool et la nicotine, il ne subsiste plus de syndrome d'abstinence, i.e. d'anxiogénèse. La Buspirone inhibe uniquement de façon significative quelques paramètres de réponse anxiogénique après l'arrêt du traitement avec l'alcool.

L'Ipsapirone maintient le syndrome d'abstinence, qui se manifeste par une réponse anxiogénique induit par le Diazépam, la cocaine et la nicotine. L'Ipsapirone inhibe le syndrome d'abstinence, qui se manifeste sous la forme d'une réponse anxiogénique, induit par l'alcool.

EFFET D'UN TRAITEMENT DE DIAZEPAM A LONG TERME ET ARRET. INHIBITION DU SYNDROME D'ABSTINENCE AVEC LES PRODUITS CORRESPONDANT AUX COMPOSES 1,4 et 11.

| TRAITEMENT | REDRESSEMENTS EN 5 MIN. C | O | ACTIVITE EN 5 MIN. C | O | TEMPS EN BOITE O | Lat. C-O |
|---|---|---|---|---|---|---|
| Contrôle | 28 | 92 | 38 | 80 | 58 | 13 |
| Diazépam | 70 * | 25 * | 73 * | 24 * | 25 * | 27 * |
| Diaz. + arrêt 24 h | 5 + | 98 + | 9 + | 100 + | 75 + | 9 + |
| Diaz + arrêt 24 h + | 80 ° * | 22 ° * | 82 ° * | 25 ° * | 30 ° * | 30 ° * |
| Diaz. + arrêt 24 h + | 82 ° * | 24 ° * | 100 ° * | 28 ° * | 25 ° * | 38 ° * |
| Diaz. + arrêt 24 h + | 70 ° * | 25 ° * | 72 ° * | 27 ° * | 25 ° * | 22 ° * |
| Diaz. + arrêt 24 h + Buspirone | 9 + | 88 + | 12 + | 100 + | 72 + | 2 + |
| Diaz + arrêt 24 h + Ipsapirone | 15 + | 82 + | 17 + | 88 + | 90 + | 8 + |

* : p 0.001 (anxiolyse)
\+ : p 0.001 (anxiogénèse)
° : p 0.001 (réversion de l'anxiogénèse)
C - O : Claire - obscure

**EFFET D'UN TRAITEMENT DE COCAINE A LONG TERME ET ARRET. INHIBITION DU SYNDROME D'ABSTINENCE AVEC LES PRODUITS CORRESPONDANT AUX COMPOSES 1,4 et 11.**

| TRAITEMENT | REDRESSEMENTS EN 5 MIN. C | O | ACTIVITE EN 5 MIN. C | O | TEMPS EN BOITE O | Lat. C-O |
|---|---|---|---|---|---|---|
| Contrôle | 23 | 71 | 32 | 80 | 58 | 10 |
| Cocaïne | 60 * | 38 * | 68 * | 30 * | 30 * | 18 * |
| Coca. + arrêt 24 h | 8 + | 102 + | 10 + | 105 + | 75 + | 3 + |
| Coca. + arrêt 24 h + **Composé 1** | 60 ° * | 40 ° * | 65 ° * | 20 ° * | 30 ° * | 16 ° * |
| Coca. + arrêt 24 h + **Composé 4** | 80 ° * | 20 ° * | 85 ° * | 25 ° * | 25 ° * | 33 ° * |
| Coca. + arrêt 24 h + **Composé 11** | 5 + | 90 + | 18 + | 100 + | 70 + | 4 + |
| Coca + arrêt 24 h + Buspirone | 10 + | 98 + | 18 + | 90 + | 70 + | 2 + |
| Coca + arrêt 24 h + Ipsapirone | 11 + | 89 + | 18 + | 85 + | 62 + | 8 + |

* : p 0.001 (anxiolyse)
+ : p 0.001 (anxiogénèse)
° : p 0.001 (réversion de l'anxiogénèse)
C - O : Claire - obscure

**EFFET D'UN TRAITEMENT D'ALCOOL A LONG TERME ET ARRET. INHIBITION DU SYNDROME D'ABSTINENCE AVEC LES PRODUITS CORRESPONDANT AUX COMPOSES 1,4 et 11.**

| TRAITEMENT | REDRESSEMENTS EN 5 MIN. C | O | ACTIVITE EN 5 MIN. C | O | TEMPS EN BOITE O | Lat. C-O |
|---|---|---|---|---|---|---|
| Contrôle | 23 | 72 | 37 | 76 | 58 | 13 |
| Alcool | 72 * | 22 * | 80 * | 28 * | 28 * | 28 * |
| Alcool + arrêt 24 h | 8 + | 98 + | 10 + | 108 + | 70 + | 4 + |
| Alcool + arrêt 24 h + Composé 1 | 85 ° * | 21 ° * | 85 ° * | 28 ° * | 25 ° * | 32 ° * |
| Alcool + arrêt 24 h + Composé 4 | 85 ° * | 20 ° * | 92 ° * | 18 ° * | 25 ° * | 40 ° * |
| Alcool + arrêt 24 h + Composé 11 | 55 ° * | 35 ° * | 60 ° * | 40 ° * | 28 ° * | 19 ° * |
| Alcool + arrêt 24 h + Buspirone | 18 ° | 80 ° | 15 | 75 ° | 62 | 7 |
| Alcool + arrêt 24 h + Ipsapirone | 21 ° | 75 ° | 36 ° | 80 ° | 35 ° | 10 ° |

* : p 0.001 (anxiolyse)
+ : p 0.001 (anxiogénèse)
° : p 0.001 (réversion de l'anxiogénèse
C - O : Claire - obscure

**EFFET D'UN TRAITEMENT DE NICOTINE A LONG TERME ET ARRET. INHIBITION DU SYNDROME D'ABSTINENCE AVEC LES PRODUITS CORRESPONDANT AUX COMPOSES 1,4 et 11.**

| Traitement | Redressements en 5 min. C | O | Activité en 5 min. C | O | Temps en boite obscure | Lat. C-O |
|---|---|---|---|---|---|---|
| Contrôle | 22 | 70 | 30 | 75 | 57 | 12 |
| Nicotine | 66 * | 23 * | 70 * | 25 * | 25 * | 26 * |
| Nic. + arrêt 24 h | 10 + | 96 + | 12 + | 105 + | 70 + | 4 + |
| Nic. + arrêt 24 h + Composé 1 | 75 o * | 22 o * | 65 o * | 20 o * | 23 o * | 28 o * |
| Nic. + arrêt 24 h + Composé 4 | 78 o * | 20 o * | 90 o * | 22 o * | 23 o * | 42 o * |
| Nic. + arrêt 24 h + Composé 11 | 28 o | 68 o | 40 o | 68 o | 30 o | 15 o |
| Nic. + arrêt 24 h + Buspirone | 12 | 80 | 20 | 85 | 65 | 8 |
| Nic. + arrêt 24 h + Ipsapirone | 9 + | 84 + | 18 + | 98 + | 70 + | 3 + |

* : p 0.001 (anxiolyse)
+ : p 0.001 (anxiogénèse)
o : p 0.001 (réversion de l'anxiogénèse)
C - O : Claire - Obscure

Les dérivés de formule générale I selon l'invention sont donc utiles comme substances actives de médicaments destinés au traitement de troubles associés au syndrome d'abstinence qui se manifeste en particulier sous la forme d'une réponse anxiogénique, induit par la suppression brusque d'un traitement prolongé avec des benzodiazépines telles que le Diazépam, de la cocaïne ou d'une absorption prolongée d'alcool et/ou de nicotine.

En thérapeutique humaine, la dose d'administration est bien sûr fonction de la gravité du syndrome.

Elle sera généralement comprise entre environ 5 et environ 100 mg/jour.

Les dérivés de l'invention seront, par exemple, administrés sous forme de comprimés, de solutions ou de

suspensions, ou bien de gélules.

On indiquera, ci-après, à titre d'exemples, deux formes galéniques particulières.

**Exemple de formule par comprimé**

| | |
|---|---|
| Composé 1 | 5 mg |
| Lactose | 60 mg |
| Cellulose microcristalline | 25 mg |
| Povidone | 5 mg |
| Amidon prégélatinisé | 3 mg |
| Dioxyde de silice colloïdale | 1 mg |
| Stéarate de magnésium | 1 mg |
| Poids comprimé | 100 mg |

**Exemple de formule par gélule**

| | |
|---|---|
| Composé 4 | 10 mg |
| Glycérine polyoxyéthylénée | 135 mg |
| Behénate de glycérine | 5 mg |
| | 150 mg |

Excipient : gélatine molle q.s

**Revendications**

1. Utilisation des dérivés de formule générale I

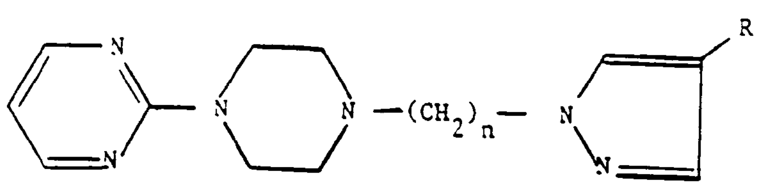

(I)

dans laquelle :

n peut avoir les valeurs 1 à 6, et

R représente un atome d'hydrogène, un halogène, un radical alkyle inférieur en $C_1$ à $C_4$, un radical pyrolyle, un radical sulfonique, un radical N-diméthyl-sulfamoyle, un radical nitro, un radical alcoxy inférieur en $C_1$ à $C_4$, un radical cyano, un radical carboxylate d'alkyle inférieur en $C_1$ à $C_4$, un radical phényle ou phényle substitué par un atome de chlore ou par un groupe méthoxy, un radical amino ou amino substitué, de formule

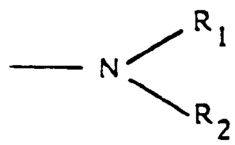

dans laquelle
$R_1$et $R_2$, identiques ou différents, représentent un atome d'hydrogène, un radical alkyle, un radical alkylcarboxy, un radical phénylcarboxy, un radical alkylsulfonyle, un radical phénylsulfonyle ou un radical tolylsulfonyle, les fragments alkyle de ces radicaux contenant de 1 à 4 atomes de carbone,
et leurs sels thérapeutiquement acceptables,
pour la fabrication de médicaments destinés au traitement des troubles associés au syndrome d'abstinence induit par la suppression de benzodiazépines telles que le Diazépam, de la cocaïne, de l'alcool et/ou de la nicotine.

2. Utilisation selon la revendication 1, caractérisée en ce que le dérivé de formule générale I est choisi parmi :
1H-pyrazole-1-(4-(4-(2-pyrimidinyl)-1-pipérazinyl)-butyl),
1H-pyrazole-4-méthyl-1-(4-(4-(2-pyrimidinyl)-1-pipérazinyl)-butyl),
1H-pyrazole-4-nitro-1-(4-(4-(2-pyrimidinyl)-1-pipérazinyl)-butyl),
1H-pyrazole-4-chloro-1-(4-(4-(2-pyrimidinyl)-1-pipérazinyl)-butyl),
1H-pyrazole-4-carboxylate d'éthyle-1-(4-(4-(2-pyrimidinyl)-1-pipérazinyl)-butyl),
1H-pyrazole-4-bromo-1-(4-(4-(2-pyrimidinyl)-1-pipérazinyl)-butyl),
1H-pyrazole-4-cyano-1-(4-(4-(2-pyrimidinyl)-1-pipérazinyl)-butyl),
1H-pyrazole-4-fluoro-1-(4-(4-(2-pyrimidinyl)-1-pipérazinyl)-butyl),
1H-pyrazole-4-méthoxy-1-(4-(4-(2-pyrimidinyl)-1-pipérazinyl)-butyl),
1H-pyrazole-4-amino-1-(4-(4-(2-pyrimidinyl)-1-pipérazinyl)-butyl),
1H-pyrazole-4-méthylsulfonamido-1-(4-(4-(2-pyrimidinyl)-1-pipérazinyl)-butyl),
1H-pyrazole-4-benzamido-1-(4-(4-(2-pyrimidinyl)-1-pipérazinyl)-butyl),
1H-pyrazole-4-acétamido-1-(4-(4-(2-pyrimidinyl)-1-pipérazinyl)-butyl),
1H-pyrazole-4-(2-butyl)amino-1-(4-(4-(2-pyrimidinyl)-1-pipérazinyl)-butyl).
1H-pyrazole-4-(4-méthoxyphényl)-1-(4-(4-(2-pyrimidinyl)-1-pipérazinyl)-butyl),
1H-pyrazole-4-(4-chlorophényl)-1-(4-(4-(2-pyrimidinyl)-1-pipérazinyl)-butyl),
1H-pyrazole-4-(1-pyrrolyl)-1-(4-(4-(2-pyrimidinyl)-1-pipérazinyl)-butyl),
1H-pyrazole-4-phényl-1-(4-(4-(2-pyrimidinyl)-1-pipérazinyl)-butyl),
1H-pyrazole-4-phénylsulfonamido-1-(4-(4-(2-pyrimidinyl)-1-pipérazinyl)-butyl),
1H-pyrazole-4-(4-méthylbenzene)sulfonamido-1-(4-(4-(2-pyrimidinyl)-1-pipérazinyl)-butyl),
1H-pyrazole-4-butylsulfonamido-1-(4-(4-(2-pyrimidinyl)-1-pipérazinyl)butyl),
1H-pyrazole-4-propylsulfonamido-1-(4-(4-(2-pyrimidinyl)-1-pipérazinyl)-butyl),
1H-pyrazole-4-éthylsulfonamido-1-(4-(4-(2-pyrimidinyl)-1-pipérazinyl)-butyl),
1H-pyrazole-4-N-diméthyl-sulfamoyle-1-(4-(4-(2-pyrimidinyl)-1-pipérazinyl)-butyl),
1H-pyrazole-4-sulfonique-1-(4-(4-(2-pyrimidinyl)-1-pipérazinyl)-butyl),
1H-pyrazole-4-chloro-1-(4-(4-(2-pyrimidinyl)-1-pipérazinyl)-butyl)hydrochlorure,
1H-pyrazole-4-chloro-1-(4-(4-(2-pyrimidinyl)-1-pipérazinyl)-butyl)dihidrochlorure,

## Claims

1. Use of the derivatives of general formula I

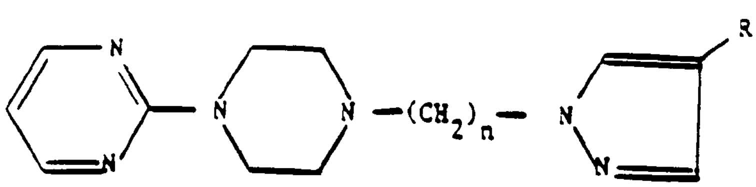

(I)

in which:

n can have the values 1 to 6, and

R represents a hydrogen atom, a halogen, a $C_1$ to $C_4$ lower alkyl radical,, a N-dimethyl-sulfamoyl radical, a nitro radical, a $C_1$ to $C_4$ lower alkoxy radical, a cyano radical, a $C_1$ to $C_4$ lower alkyl carboxylate radical, a phenyl radical or a phenyl radical substituted with a chlorine atom or a methoxy group or an amino or substituted amino radical of formula

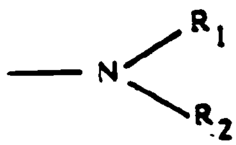

in which

$R_1$ and $R_2$, which May be identical or different, represent a hydrogen atom, an alkyl radical, an alkylcarboxy radical, a phenylcarboxy radical, an alkylsulphonyl radical or a phenylsulphonyl radical, the alkyl fragments of these radicals containing from 1 to 4 carbon atoms,

and their therapeutically acceptable salts,

for the manufacture of medicinal products intended for the treatment of disorders associated with the withdrawal syndrome induced by the discontinuation of benzodiazepines such as diazepam, cocaine, alcohol and/or nicotine.

2. Use according to Claim 1, characterized in that the derivative of general formula I is selected from:
1-{4-[4-(2-pyrimidinyl)-1-piperazinyl]butyl}-1H- pyrazole,
4-methyl-1-1-{4-[4-(2-pyrimidinyl)-1-piperazinyl]-butyl}-1H-pyrazole,
4-nitro-1-{4-[4-(2-pyrimidinyl)-1-piperazinyl]-butyl}-1H-pyrazole,
4-chloro-1-{4-[4-(2-pyrimidinyl)-1-piperazinyl]-butyl}-1H-pyrazole,
4-ethoxycarbonyl-1-{4-[4-(2-pyrimidinyl)-1-piperazinyl]-butyl}-1H-pyrazole,
4-bromo-1-{4-[4-(2-pyrimidinyl)-1-piperazinyl]-butyl}-1H-pyrazole,
4-cyano-1-{4-[4-(2-pyrimidinyl)-1-piperazinyl]-butyl}-1H-pyrazole,
4-fluoro-1-{4-[4-(2-pyrimidinyl)-1-piperazinyl]-butyl}-1H-pyrazole,
4-methoxy-1-{4-[4-(2-pyrimidinyl)-1-piperazinyl]-butyl}-1H-pyrazole,
4-amino-1-{4-[4-(2-pyrimidinyl)-1-piperazinyl]-butyl}-1H-pyrazole,
4-methylsulphonamido-1-{4-[4-(2-pyrimidinyl)-1-piperazinyl]-butyl}-1H-pyrazole,
4-benzamido-1-{4-[4-(2-pyrimidinyl)-1-piperazinyl]-butyl}-1H-pyrazole,
4-acetamido-1-{4-[4-(2-pyrimidinyl)-1-piperazinyl]-butyl}-1H-pyrazole,
4-(2-butyl)amino-1-{4-[4-(2-pyrimidinyl)-1-piperazinyl]-butyl}-1H-pyrazole,
4-(4-methoxyphenyl)-1-{4-[4-(2-pyrimidinyl)-1-piperazinyl]-butyl}-1H-pyrazole,
4-(4-chlorophenyl)-1-{4-[4-(2-pyrimidinyl)-1-piperazinyl]-butyl}-1H-pyrazole,
4-(1-pyrrolyl)-1-{4-[4-(2-pyrimidinyl)-1-piperazinyl]-butyl}-1H-pyrazole,
4-phenyl-1-{4-[4-(2-pyrimidinyl)-1-piperazinyl]-butyl}-1H-pyrazole,
4-phenylsulphonamido-1-{4-[4-(2-pyrimidinyl)-1-piperazinyl]butyl}-1H-pyrazole,
4-(4-methylbenzenesulphonamido)-1-{4-[4-(2-pyrimidinyl)-1-piperazinyl]butyl}-1H-pyrazole,
4-butylsulphonamido-1-{4-[4-(2-pyrimidinyl)-1-piperazinyl]butyl}-1H-pyrazole,
4-propylsulphonamido-1-{4-[4-(2-pyrimidinyl)-1-piperazinyl]butyl}-1H-pyrazole,
4-ethysulphonamido-1-{4-[4-(2-pyrimidinyl)-1-piperazinyl]butyl}-1H-pyrazole,
4-(N,N-dimethylsulphamoyl)-1-{4-[4-(2-pyrimidinyl)-1-piperazinyl]butyl}-1H-pyrazole,
4-sulpho-1-{4-[4-(2-pyrimidinyl)-1-piperazinyl]butyl}-1H-pyrazole,
4-chloro-1-{4-[4-(2-pyrimidinyl)-1-piperazinyl]butyl}-1H-pyrazole hydrochloride,
4-chloro-1-{4-[4-(2-pyrimidinyl)-1-piperazinyl]butyl}-1H-pyrazole dihydrochloride.

## Patentansprüche

1. Verwendung von Derivaten der allgemeinen Formel (I)

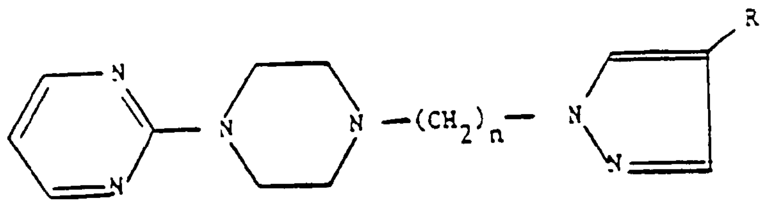

(I)

in der

n die Werte 1 bis 6 haben kann und

R für ein Wasserstoffatom, ein Halogenatom, einen niederen Alkylrest mit 1 bis 4 Kohlenstoffatomen, einen Pyrrolrest, einen Sulfonsäurerest, einen N-Dimethylsulfamoyl-Rest, einen Nitrorest, einen niederen Alkoxyrest mit 1 bis 4 Kohlenstoffatomen, einen Cyanorest, einen niederen Alkylcarboxylatrest mit 1 bis 4 Kohlenstoffatomen, einen Phenylrest oder einen durch ein Chloratom oder durch eine Methoxygruppe substituierten Phenylrest, einen Aminorest oder einen substituierten Aminorest

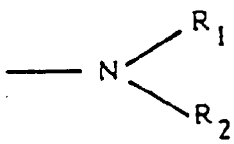

worin $R_1$ und $R_2$, die identisch oder verschieden sind, ein Wasserstoffatom, einen Alkylrest, einen Alkylcarboxyrest, einen Phenylcarboxyrest, einen Alkylsulfonylrest, einen Phenylsulfonylrest oder einen Tolylsulfonylrest darstellen, wobei die Alkylgruppen dieser Reste 1 bis 4 Kohlenstoffatome enthalten, und ihrer therapeutisch akzeptablen Salze zur Herstellung von Arzneimitteln für die Behandlung von Störungen, die mit dem Entzugssyndrom bei Kokain, Alkohol und/oder Nikotin in Zusammenhang stehen, das hervorgerufen wird durch die Unterdrückung von Benzodiazepinen wie Diazepam.

2. Verwendung nach Anspruch 1, dadurch gekennzeichnet, daß das Derivat der allgemeinen Formel I ausgewählt wird aus:
1H-Pyrazol-1-(4-(4-(2-pyrimidinyl)-1-piperazinyl)butyl,
1H-Pyrazol-4-methyl-1-(4-(4-(2-pyrimidinyl)-l-piperazinyl)butyl),
1H-Pyrazol-4-nitro-1-(4-(4-(2-pyrimidinyl)-1-piperazinyl)butyl),
1H-Pyrazol-4-chloro-1-(4-(4-(2-pyrimidinyl)-1-piperazinyl)butyl),
Ethyl-1-(4-(4-(2-pyrimidinyl)-1-piperazinyl)-butyl-1H-pyrazol-4-carboxylat,
1H-Pyrazol-4-bromo-1-(4-(4-(2-pyrimidinyl)-1-piperazinyl)butyl),
1H-Pyrazol-4-cyano-1-(4-(4-(2-pyrimidinyl)-1-piperazinyl)butyl),
1H-Pyrazol-4-fluoro-1-(4-(4-(2-pyrimidinyl)-1-piperazinyl)-butyl),
1H-Pyrazol-4-methoxy-1-(4-(4-(2-pyrimidinyl)-1-piperazinyl)-butyl,
1H-Pyrazol-4-amino-1-(4-(4-(2-pyrimidinyl)-1-piperazinyl)butyl),
1H-Pyrazol-4-methylsulfonamido-1-(4-(4-(2-pyrimidinyl)-1-piperazinyl)-butyl),
1H-Pyrazol-4-benzamido-1-(4-(4-(2-pyrimidinyl)-1-piperazinyl)-butyl),
1H-Pyrazol-4-acetamido-1-(4-(4-(2-pyrimidinyl)-1-piperazinyl)-butyl),
1H-Pyrazol-4-(2-butyl)amino-1-(4-(4-(2-pyrimidinyl)-1-piperazinyl)-butyl),
1H-Pyrazol-4-(4-methoxyphenyl)-1-(4-(4-(2-pyrimidinyl)-1-piperazinyl)-butyl),
1H-Pyrazol-4-(4-chlorophenyl)-1-(4-(4-(2-pyrimidinyl)-1-piperazinyl)-butyl),
1H-Pyrazol-4-(1-pyrrolyl)-1-(4-(4-(2-pyrimidinyl)-1-piperazinyl)-butyl),
1H-Pyrazol-4-phenyl-1-(4-(4-(2-pyrimidinyl)-1-piperazinyl)-butyl),
1H-Pyrazol-4-phenylsulfonamido-1-(4-(4-(2-pyrimidinyl)-1-piperazinyl)-butyl),
1H-Pyrazol-4-(4-methylbenzol)sulfonamido-1-(4-(4-(2-pyrimidinyl)-1-piperazinyl)-butyl),
1H-Pyrazol-4-butylsulfonamido-1-(4-(4-(2-pyrimidinyl)-1-piperazinyl)-butyl),
1H-Pyrazol-4-propylsulfonamido-1-(4-(4-(2-pyrimidinyl)-1-piperazinyl)-butyl),
1H-Pyrazol-4-ethylsulfonamido-1-(4-(4-(2-pyrimidinyl)-1-piperazinyl)-butyl),

1H-Pyrazol-4-N-dimethylsulfamoyl-1-(4-(4-(2-pyrimidinyl-1-piperazinyl)-butyl),
1H-Pyrazol-4-sulfonsäure-1-(4-(4-(2-pyrimidinyl)-1-piperazinyl)-butyl),
1H-Pyrazol-4-chloro-1-(4-(4-(2-pyrimidinyl)-1-piperazinyl)-butyl)hydrochlorid,
1H-Pyrazol-4-chloro-1-(4-(4-(2-pyrimidinyl)-1-piperazinyl)-butyl)dihydrochlorid.